# EUROPEAN PATENT APPLICATION

(11) **EP 3 412 197 A1**
(43) Date of publication of application: **12.12.2018**
(21) Application number: 18172608.4
(22) Date of filing: 16.05.2018
(51) Int. Cl.: A61B 1/24, A61B 1/00, A61C 9/00

(54) **CALIBRATION CRADLE FOR INTRAORAL SCANNER AND INTRAORAL SCANNER SYSTEM INCLUDING THE SAME**

(30) Priority: 17.05.2017 KR 20170060818
(71) Applicant: Vatech Co., Ltd., Hwaseong-si, Gyeonggi-do 18449 (KR); Vatech Ewoo Holdings Co. Ltd., Hwaseong-si, Gyeonggi-do 18449 (KR)
(72) Inventor: Lee, Jung, Seoul 07326 (KR)
(74) Representative: Markfort, Iris-Anne Lucie

(57) **Abstract**

Disclosed is a cradle for an intraoral scanner which is configured to eliminate a risk of losing a calibration tool and to accurately calibrate the intraoral scanner irrespective of stability or proficiency of a user's operation. A calibration cradle for an intraoral scanner according to the present invention includes a supporting unit supporting the intraoral scanner including a body and a probe tip provided with a scan hole, and a calibration unit including a reference surface provided at a position facing the scan hole and having at least one of a color and a pattern that are formed for calibration of the intraoral scanner.

## Description

### BACKGROUND OF THE INVENTION

### Field of the Invention

The present invention relates generally to a three-dimensional scanner system. More particularly, the present invention relates to an intraoral scanner configured to insert a probe tip thereof into an oral cavity of an examinee and obtain multiple optical images so as to generate three-dimensional model data for an intraoral structure, and a cradle for mounting the intraoral scanner.

### Description of the Related Art

In general, an intraoral scanner is a type of three-dimensional scanner that obtains multiple optical images of a target object through a series of scanning sequences and generates three-dimensional model data for the target object using the obtained optical images. Of three-dimensional scanners, the intraoral scanner denotes an apparatus configured to obtain a series of optical images of a part of a body, particularly an intraoral structure such as teeth, gums, etc.

As an example of a method of obtaining an optical image using the intraoral scanner so as to provide the three-dimensional model data, there is a method, so-called active stereo vision, in which known patterned light is projected onto a tooth surface and a projected pattern is captured by at least one optical camera whereby multiple optical images are obtained. Another example is a triangulation method in which structural light is projected onto a tooth surface and an image thereof is captured by at least one optical camera whereby multiple optical images are obtained.

Either of the above two methods may cause errors in the 3D model data during a manufacturing process of the intraoral scanner or during use thereof. In a case where a portion of the intraoral scanner, that is, a probe tip having an optical element such as a reflector, etc. is configured to be replaceable for hygienic purposes, the replaceable probe tip may also be a source of error. Thus, an error correction operation, that is, calibration, for the intraoral scanner is often required in order to obtain accurate three-dimensional model data. For this reason, it is common for the intraoral scanner to be provided with a calibration tool as a separate accessory.

However, the calibration tool provided as the accessory to the intraoral scanner in the related art is liable to be lost because stable mounting thereof is impossible. In addition, a user manipulates the intraoral scanner and the calibration tool while holding them together by hand to perform the calibration, so that it is difficult to precisely perform the calibration due to a clearance between the intraoral scanner and the calibration tool, vibration caused by hand movement, etc.

### Documents of Related Art

### Patent Document

(Patent Document 1) Korean Patent Application Publication No. 10-2015-0082438 (published on July 15, 2015)

### SUMMARY OF THE INVENTION

Accordingly, in considering the prior art, an object of the present invention is to provide a cradle for an intraoral scanner which is configured to eliminate a risk of losing a calibration tool and to accurately calibrate the intraoral scanner irrespective of stability or proficiency of a user's operation, and an intraoral scanner system including the same.

In order to accomplish the above object, the present invention provides a calibration cradle for an intraoral scanner, the calibration cradle including: a supporting unit supporting the intraoral scanner including a body and a probe tip provided with a scan hole; and a calibration unit including a reference surface provided at a position facing the scan hole and having at least one of a color and a pattern that are formed for calibration of the intraoral scanner.

The calibration cradle may further include a driving unit driving the calibration unit so as to change at least one of an angle and a spacing of the reference surface relative to the scan hole.

The calibration cradle may further include a cradle case having the supporting unit formed at a side thereof and accommodating therein at least a portion of the calibration unit including the reference surface.

The cradle case may include a probe tip accommodating unit positioning the scan hole and the reference surface to face each other with a space being defined between the scan hole and the reference surface.

The calibration cradle may further include a cradle controller connected with the intraoral scanner wired or wirelessly and controlling the intraoral scanner to perform the calibration.

The cradle controller may include: a counter counting elapsed time from the most recent calibration time; and a calibration execution unit controlling the intraoral scanner such that when the elapsed time reaches a preset calibration period, the calibration is performed.

The cradle controller may further include a period setting unit setting the calibration period according to user input.

Meanwhile, the calibration cradle may further include a cradle controller connected with the intraoral scanner wired or wirelessly and controlling the intraoral scanner and the driving unit to perform the calibration.

Herein, the cradle controller may include: a counter counting elapsed time from the most recent calibration time; and a calibration execution unit controlling the intraoral scanner such that when the elapsed time reaches a predetermined calibration period, the calibration is performed.

According to an aspect of the present invention, there is provided an intraoral scanner system, including: an intraoral scanner including a body and a probe tip provided with a scan hole; and a calibration cradle supporting the intraoral scanner and including a reference surface provided for calibration at a position facing the scan hole.

The calibration cradle may further include a cradle controller connected with the intraoral scanner wired or wirelessly and controlling the intraoral scanner to perform the calibration.

The calibration cradle may further include a driving unit driving a calibration unit so as to change at least one of an angle and a spacing of the reference surface relative to the scan hole.

In this case, the calibration cradle may further include a cradle controller connected with the intraoral scanner wired or wirelessly and controlling the intraoral scanner and the driving unit to perform the calibration.

According to the present invention, the calibration cradle for the intraoral scanner having a calibration function is provided, whereby there is no risk of losing the calibration tool, and the intraoral scanner can be accurately calibrated irrespective of stability or proficiency of a user's operation. As a result, it is possible to increase accuracy of three-dimensional model data generated by the intraoral scanner and to improve convenience of maintenance of the intraoral scanner.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 is a view showing an intraoral scanner mounted on a calibration cradle according to an embodiment of the present invention;
FIG. 2 is a view showing calibration of the intraoral scanner using the calibration cradle according to the embodiment of the present invention;
FIG. 3 is a detailed view showing a configuration example of a calibration unit in the embodiment of FIG. 2;
FIG. 4 is a view showing a calibration execution sequence according to an aspect of the present invention;
FIG. 5 is a block diagram showing a schematic configuration of the calibration cradle according to the embodiment of the present invention; and
FIG. 6 is a block diagram showing a schematic configuration of the intraoral scanner according to the embodiment of the present invention.

### DETAILED DESCRIPTION OF THE INVENTION

Hereinbelow, exemplary embodiments of the present invention will be described in detail with reference to the accompanying drawings. The exemplary embodiments described hereinbelow are provided for fully conveying the scope and spirit of the invention to those skilled in the art, so it should be understood that the scope and spirit of the invention are not limited to the embodiments described hereinbelow. Throughout the drawings, the same reference numerals will refer to the same or like parts, and a description of components having the same reference numerals as those described in any one of the drawings may be omitted.

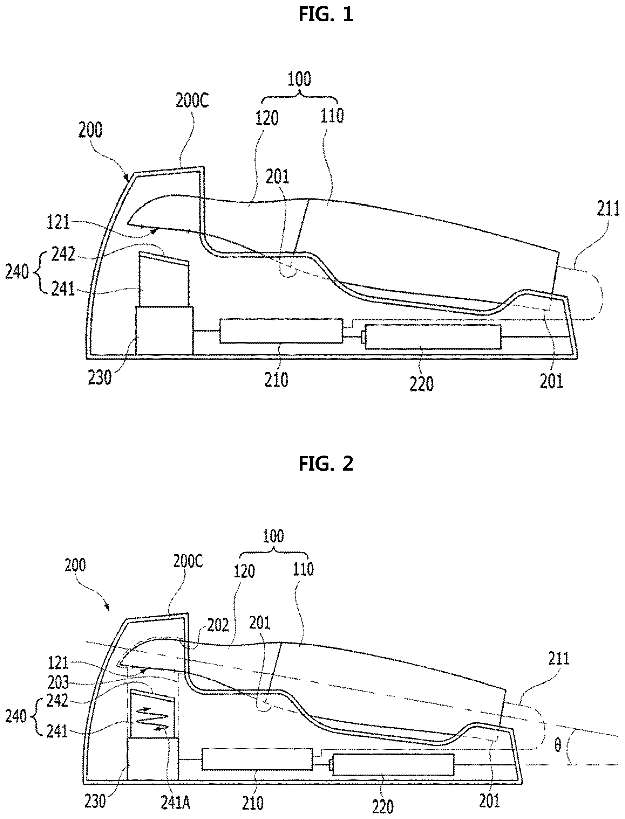

FIG. 1 is a view showing an intraoral scanner mounted on a calibration cradle according to an embodiment of the present invention.

First, according to the embodiment of the present invention, an intraoral scanner system includes an intraoral scanner 100 configured with a main body 110 and a probe tip 120 that are detachably coupled to each other and a calibration cradle 200 which is configured to perform a calibration in a state in which the intraoral scanner 100 is mounted thereon.

Herein, the calibration cradle 200 includes: at least one supporting unit 201 supporting the intraoral scanner 100 such that the intraoral scanner 100 is supported and held with respect to a bottom surface of the calibration cradle 200 in a state of the intraoral scanner 100 is mounted therein; a calibration unit 240 including a reference surface 242 disposed at a position facing a scan hole 121 of the probe tip 120 and which may rotate and move vertically with a spiral motion. The scan hole may include a window through which projection light and reflected light of the probe tip 120 pass. A driving unit 230 providing power for driving the calibration unit 240 in response to a control signal from a cradle controller 210.

A cradle case 200C that defines a body shape of the calibration cradle 200 has the supporting unit 201 formed at the outside of the cradle case 200C and accommodates therein the calibration unit 240 and the driving unit 230. In addition, the cradle case 200C also accommodates therein the cradle controller 210 controlling the driving unit 230, and a power supply unit 220 supplying electric power thereto. For reference, although a battery is shown as the power supply unit 220 in the drawings, the power supply unit 220 may supply DC or AC electric power from an external power source.

Herein, the cradle controller 210 may be connected with the body 110 of the intraoral scanner 100 through a wired or wireless communication channel 211 to transmit a control signal.

The calibration unit 240 includes the reference surface 242 and a spiral motion unit 241 to rotate and/or move vertically the reference surface 242. The reference surface 242 may become a reference for a correction operation with the size, inclination, pattern formed thereon, while it is moved on the spiral motion unit. The driving unit 230 may include a motor that rotates the spiral motion unit 241. Herein, the reference surface 242 may have at least one of a predetermined pattern and a color, and at least one of the pattern and the color may be appropriately selected according to a manner of obtaining an optical image of the intraoral scanner.

For reference, the predetermined pattern may be formed in various shapes. For example, the pattern may be formed in a grid such that the light and shade between each of sections of the grid and a neighboring section, which are adjacent vertically and horizontally to each other, are clearly distinguished. The principle and method of performing calibration of the intraoral scanner using a calibration pattern of which an initial position, an angle, and a range of a change in position may be known in prior arts.

Furthermore, in the intraoral scanner system according to the present invention, the intraoral scanner may include a color image sensor so as to obtain an optical image of the surface of an intraoral structure to obtain information of the texture or color thereof as well as to obtain information of a three-dimensional shape of the intraoral structure to generate a model thereof. However, in this case, the color information obtained by the color image sensor may vary depending on color distribution of illumination, so it is necessary to adjust white balance to obtain color information close to the color of the teeth actually observed with the naked eye. For this purpose, the reference surface may represent a predetermined color, that is, a white color. Hereinafter, a case where the predetermined pattern is provided on the reference surface will be described for the sake of convenience.

FIG. 2 is a view showing calibration of the intraoral scanner using the calibration cradle according to the embodiment of the present invention.

In the calibration cradle 200, the cradle case 200C has a probe tip accommodating unit 202 accommodating at least a portion of the probe tip 120, more specifically, a front end portion thereof having the scan hole 121, and positioning the scan hole 121 of the accommodated probe tip 120 and the reference surface 242 of the calibration unit 240 to face each other while it is open therebetween. The probe tip accommodating unit 202 may be recessed inwardly from a outer case thereof so as to block external light for stably supporting and holding the front end portion of the probe tip 120. The probe tip accommodating unit 202 may be connected through an empty space with a calibration unit accommodating portion 203. This empty space is for vertically moving the reference surface 242, and a path through which light passes between the scan hole 121 and the reference surface 242.

The supporting unit 201 allows the intraoral scanner 100 to be supported in an inclined posture such that the probe tip 120 of the intraoral scanner 100 is relatively higher with respect to the bottom surface of the cradle case 200C. Herein, an inclination angle θ of the intraoral scanner 100 may be 8 to 16 degrees with respect to the bottom surface of the cradle case 200C. As a more specific example, the inclination angle θ may be about 12 degrees. This range of inclination angle allows a user to easily mount and dismount the intraoral scanner 100 with respect to the cradle 200, which is advantageous in terms of ergonomics, and is also suitable for ensuring a space for the calibration unit 240 and the driving unit 230 to be installed under the front end portion of the probe tip 120, which is advantageous in terms of space utilization.

A calibration process will be described with reference to an example. The cradle controller 210 transmits a control signal to the intraoral scanner 100 through the communication channel 211 to activate a calibration mode of the intraoral scanner 100 while transmitting a driving signal and electric power to the driving unit 230. As a result, when the spiral motion unit 241 start to move in a spiral motion in a direction of a spiral arrow 241A shown in the drawing, the reference surface 242 that is disposed obliquely with respect to an axis of the spiral motion is rotated and moved vertically simultaneously. In this process, the angle, the height, etc. of the reference surface 242 may be controlled with preset values by the cradle controller 210. With this operation, the measured values thereof may be provided to the cradle controller 210. A body control unit mounted in the body 110 of the intraoral scanner 100 or a system control unit connected to the body 110 from outside thereof through the communication channel performs calibration of the intraoral scanner 100 based on preset information of the angle, the height, the pattern of the reference surface 242, etc. by comparing measured information.

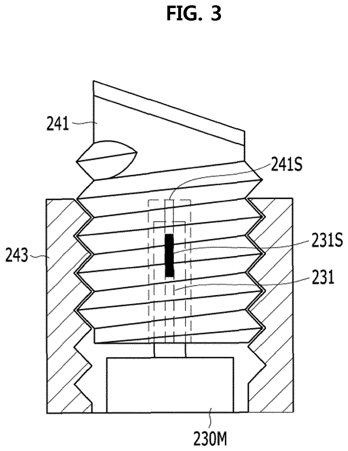

FIG. 3 is a detailed view showing a configuration example of a calibration unit in the embodiment of FIG. 2.

The cradle controller may be configured with, for example, a step motor 230M capable of controlling a rotation angle thereof. A drive shaft 231 of the motor 230M may be connected with the spiral motion unit 241 while it is sliding in an rotation axis direction of the spiral motion unit 241 and to transmit only a rotational motion thereto. For example, when a protrusion 231S is formed on a side of the drive shaft 231 and the protrusion 231S is inserted into a guide groove 241S formed along the rotation axis direction of the spiral motion unit 241, the spiral motion unit 241 is rotated and moved vertically by the relative spiral motion between the spiral motion unit 241 and a spiral fixture 243 which guides the spiral motion unit 241. However, the configuration in which the spiral motion unit 241 performs simultaneously a rotational motion and a vertical motion in cooperation with a rotational motion of the motor may be realized in various other forms.

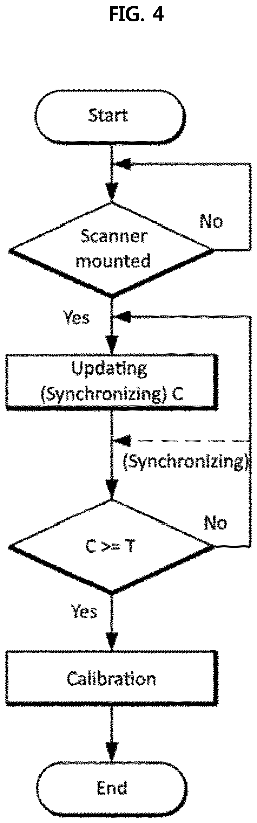

FIG. 4 is a view showing a calibration execution sequence according to an aspect of the present invention

This embodiment relates to a method of automatically calibrating the intraoral scanner every preset period (time period) by the calibration cradle in the intraoral scanner system, and also relates to control by a controller of the calibration cradle, that is, the cradle controller.

First, the cradle controller detects whether the intraoral scanner is mounted on the cradle in response to a signal from a sensor such as a pressure sensor installed in the cradle or a sensor such as an acceleration sensor installed in the body of the intraoral scanner. When the intraoral scanner is not mounted on the cradle, the cradle controller may detect the signal of the sensor at a predetermined time interval.

When it is detected that the intraoral scanner is mounted on the cradle, the intraoral scanner updates elapsed time information C indicating how much time has elapsed since completion of a previous calibration. The elapsed time information C may be stored in the body of the intraoral scanner. If the elapsed time information C is stored in the body of the intraoral scanner, the elapsed time information C may be synchronized with information in the cradle. However, the time of updating most recent calibration time information or the elapsed time information is not limited to a case where the intraoral scanner is mounted on the cradle.

When the updated or synchronized elapsed time information C is compared with a calibration period T that is preset. If the elapsed time information is equal to or greater than the calibration period T, the body and the cradle controller are driven to perform calibration as described above with reference to FIG. 2. If the elapsed time information C is less than the calibration period T, the elapsed time information of the intraoral scanner may be updated continuously at a predetermined time interval, or the elapsed time information C which synchronized the information in the cradle may be synchronized with the information in the cradle. And it may be continuously counted and accumulated, and compared again with the calibration period T.

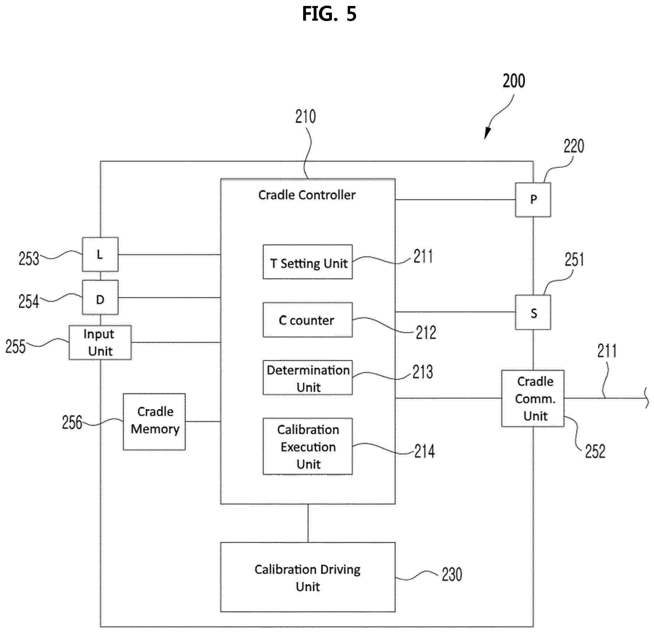

FIG. 5 is a block diagram showing a schematic configuration of the calibration cradle according to the embodiment of the present invention.

As an example of a configuration for realizing calibration operation described above, the cradle controller 210 may include a period setting unit 211 presetting a calibration period T, a counter 212 accumulatively counting elapsed time after updating the elapsed time information described above, a determination unit 213 determining whether calibration is needed to be executed by comparing the elapsed time information C accumulated until determination time with the calibration period T, and a calibration execution unit 214 generating a control signal for executing calibration according to the determination.

The cradle 200 may be provided with a cradle communication unit 252 transmitting the control signal generated by the cradle controller 210 to the intraoral scanner 100 through the communication channel 211 or receives the information described above from the intraoral scanner 100. The cradle 200 may include the supporting unit 201 supporting the intraoral scanner 100 at a side of the cradle with a mounting detection sensor detecting whether the intraoral scanner is mounted on the cradle or not and providing a result of detection to the cradle controller 210 in the form of an electrical signal.

The cradle 200 may include an input unit 255 receiving a user information which is including whether to calibrate the intraoral scanner or the calibration period T, and a cradle memory 256 storing information received from the cradle communication unit 252 and a set value that is processed by the period setting unit 211. In addition, there may be also included a lighting unit 253 which shows an operation state of the cradle 200, for example, whether the calibration takes place, and a display unit 254 which displays information including the operation state, the set value, and other information that are needed to be notified by a user as characters or images.

Meanwhile, the power supply unit 220 of the cradle 200 may supply electric power to the cradle controller 210, the driving unit 230. The external power source may be a battery. And it may also supply electric power to the intraoral scanner when connected to the intraoral scanner over wires.

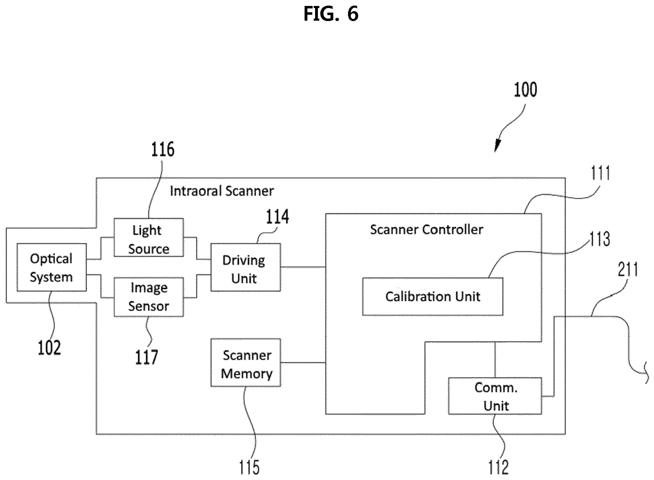

FIG. 6 is a block diagram showing a schematic configuration of the intraoral scanner according to the embodiment of the present invention

In the intraoral scanner system according to the present invention, the intraoral scanner 100 may be configured to transmit the most recent calibration time information, or the time information and the elapsed time information having elapsed therefrom to the cradle 200 through the communication channel 211 for calibration execution. Further the intraoral scanner may be configured to perform calibration by driving a light source 116 and an image sensor 117 through a driving part 114 in response to a control signal for calibration. A scanner memory 115 provided in the intraoral scanner 100 may store the most recent calibration time information, calibration history information including the most recent calibration time information, a parameter value adjusted according to a calibration result, etc.

The intraoral scanner 100 may be further configured to be controlled by a scanner controller 111. The scanner controller 111 manages a calibration operation of the intraoral scanner in response to the control signal received from the cradle controller as well as manages provision of the information through a communication unit 112. For this purpose, the scanner controller 111 includes a calibration unit 113.

Meanwhile, the light source 116 may include at least one of a patterned light, a structured light, and a light source that generates auxiliary light for illumination. Furthermore, the image sensor 117 may be one image sensor or a plurality of image sensors. Meanwhile, the intraoral scanner 100 is provided with an optical system 102 constituting a part of an optical path through which light emitted from the light source 116 is received by the image sensor 117.

On the other hand, the calibration cradle according to the embodiment of the present invention includes all the structures for supporting the intraoral scanner, and may be configured to be a portion of another device rather than a separate device.

In other words, a general intraoral scanner system includes an intraoral scanner, a computer device generating three-dimensional model data from an optical image of the intraoral scanner, and a display device on which the generated three-dimensional model data is displayed.

Herein, in order to increase mobility, at least one of the computer device and the display device may be mounted on a cart, and the intraoral scanner may be mounted on a predetermined mounting structure provided at a side of the cart. In this case, the calibration cradle according to the present invention may be implemented including a portion or the entirety of the predetermined mounting structure.

Furthermore, at least one of the computer device and the display device may be installed at a chair unit that is a chair for dental care, and the intraoral scanner may be mounted on a handpiece holder provided at the chair unit. In this case, the calibration cradle according to the present invention may be implemented including a portion or the entirety of the handpiece holder.

Although the exemplary embodiments of the present invention have been described for illustrative purposes, those skilled in the art will appreciate that various modifications, additions and substitutions are possible, without departing from the scope and spirit of the invention as disclosed in the accompanying claims.

## Claims

1. A calibration cradle for an intraoral scanner, the calibration cradle comprising:
a supporting unit supporting the intraoral scanner including a body and a probe tip provided with a scan hole; and
a calibration unit including a reference surface provided at a position facing the scan hole and having at least one of a color and a pattern that are formed for calibration of the intraoral scanner.

2. The calibration cradle of claim 1, further comprising:
a driving unit driving the calibration unit so as to change at least one of an angle and a spacing of the reference surface relative to the scan hole.

3. The calibration cradle of claim 1, further comprising:
a cradle case including the supporting unit formed at a side thereof and accommodating therein at least a portion of the calibration unit including the reference surface.

4. The calibration cradle of claim 3, wherein the cradle case includes a probe tip accommodating unit positioning the scan hole and the reference surface to face each other with a space being defined between the scan hole and the reference surface.

5. The calibration cradle of claim 1, further comprising:
a cradle controller connected with the intraoral scanner by being wired or wirelessly and controlling the intraoral scanner to perform the calibration.

6. The calibration cradle of claim 5, wherein the cradle controller includes:
a counter counting elapsed time from the most recent calibration time; and
a calibration execution unit controlling the intraoral scanner such that when the elapsed time reaches a preset calibration period, the calibration is performed.

7. The calibration cradle of claim 6, wherein the cradle controller further includes a period setting unit setting the calibration period according to user input.

8. The calibration cradle of claim 2, further comprising:
a cradle controller connected with the intraoral scanner wired or wirelessly and controlling the intraoral scanner and the driving unit to perform the calibration.

9. The calibration cradle of claim 8, wherein the cradle controller includes:
a counter counting elapsed time from the most recent calibration time; and
a calibration execution unit controlling the intraoral scanner such that when the elapsed time reaches a predetermined calibration period, the calibration is performed.
